# EUROPEAN PATENT APPLICATION

(11) **EP 4 385 410 A1**
(43) Date of publication of application: **19.06.2024**
(21) Application number: 22213154.2
(22) Date of filing: 13.12.2022
(51) Int. Cl.: A61B 5/266, A61B 5/268, A61B 5/27

(54) **CONTACT SYSTEM FOR PROVIDING ELECTRICAL CONTACT WITH AN OBJECT**

(71) Applicant: Nahtlos AG, 9014 St. Gallen (CH)
(72) Inventor: SCHMID, Michel, 9011 St. Gallen (CH); NÄF, José, 9443 Widnau (CH)
(74) Representative: Kaminski Harmann Patentanwälte AG (Schweiz)

(57) **Abstract**

The invention relates to a contact system (1) for providing an electric contact to the skin of a person, the contact system (1) comprising a reservoir (10) having a fluid (11) and a conductive layer (30). The reservoir (10) comprises a transmissive layer (12), the transmissive layer (12) being configured to provide defined transmission of vapour and/or vapour-like substance, and the transmissive layer (12) and the conductive layer (30) are arranged relative to each other so that humidifying of the conductive layer (30) by transmission of vaporous parts of the fluid through the transmissive layer (12) is provideable.

## Description

### FIELD OF THE INVENTION

The present invention relates to a contact unit, in particular an electrode, for providing an electric contact to an object, e.g. to the skin of a person, and transmission of an electrical signal from the object to a signal receiver and/or from a signal generator to the object.

### BACKGROUND OF THE INVENTION

The measurement of electrical body signals such as ECG (electrocardiogram), EEG (electroencephalogram), EMG (electromyogram) are widely used measuring methods for deriving body signals. Today the ECG is used in the medical field typically with Ag/AgCI gel electrodes, which are glued to the skin around the chest area. These electrodes are more or less a standard and are used worldwide for ECG measurements in the medical field.

A disadvantage of these electrodes is that they only work for a limited time of up to approx. 24 h or 72 h. After that they dry out and no longer provide a suitable signal. However, it has been that long-term measurements can also be essential. In that context, it can be important to monitor the ECG over several days, preferably over more than seven days, by means of special loggers. For this purpose new dry electrodes have appeared on the market, which should be suitable for ECG measurements. For example, embroidery electrodes and sensor shirts are known. Such electrodes work during physical activity and give good signals, especially when the person is sweating and thus has moist skin.

As soon as the electrodes are dry, measurement is no longer possible. The entire medical area where the skin is not moist but dry cannot be measured with these electrodes for a long time. However, this area would be interesting from a scientific and medical point of view, e.g. the monitoring of cardiac risk patients or, in general, with older people where normally dry skin is present.

Another problem with dry electrodes is the movement artifacts and the relatively high pressure of the electrode on the skin. With dry skin, movement is even more critical, i.e. the slightest movement of the arms or chest can lead to extremely high artifacts. To filter out a reasonable ECG signal from these is extremely difficult.

To overcome above disadvantages, US 5,057,072 A proposes an electrode with a reservoir containing a chemical or ionic liquid where the liquid is on the skin side and the electrode is located behind it. Here, it appears to be a disadvantage that the moistening comes from the skin side.

US 6,263,226 B1 describes a disposable electrode which is disposed of after 8 to 12 hours. This is impractical. For electrodes, continuous use for weeks is preferred. Furthermore, an electrically conductive gel is required between the skin and the actual electrical electrode. It appears disadvantageously when the (stick) electrode would not lie directly on the skin, and a gel or sponge is required between the skin and the electrode.

From WO 2004/017829 A1 electrodes are known, which are intended for impedance tomography. However, for electrocardiography, these electrodes appear unsuitable. Again, a gel is provided between the electrode and the skin, which should be avoided because of intended continuous use.

From EP 510 786 A1 an electrode is known, which is based on a hydrogel layer, which is brought together in combination with a non-conductive material layer and is connected to a reusable clamp. No moistening device is provided. The device of EP 510 786 A1 is based on the principle of an electrolyte layer based on hydrogel.

The approaches of above have in common that they can either only be used for a very limited time and/or represent comparatively complex electrodes.

### OBJECT OF THE INVENTION

Accordingly, it is an object of the invention to propose an improved contact unit in which the described disadvantages of the prior art can at least be reduced.

It is an object of the invention to provide an improved contact unit for providing an electric contact to the skin of a person.

It is a further object of the invention to provide an improved contact unit which enabled long-term measurements and which is easy to use.

These objects are achieved by realizing at least part of the features of the independent claims. Features which further develop the invention in an alternative or advantageous manner are described in the dependent patent claims.

### SUMMARY OF THE INVENTION

The present invention relates to a contact system, in particular an electrode, for providing an electric contact to the skin of a person. The contact system may also be referred to as electrode. The contact system comprises a reservoir having a fluid and a conductive layer. The reservoir comprises a transmissive layer. The transmissive layer is configured to provide defined transmission of the fluid and/or of fluid-based vapour and/or vapour-like substance and/or vaporous substance with a defined transmission rate. The transmissive layer and the conductive layer are arranged relative to each other so that humidifying of the conductive layer by transmission of vaporous parts of the fluid through the transmissive layer is provideable.

In particular, the transmissive layer is embodied to provide the defined transmission rate. The transmission rate can be adjusted to a particular fluid which is comprised by the reservoir, i.e. the transmissive layer and the fluid can be chosen or designed relative to each other so that a particular amount of the fluid and/or of the fluid-based vapour can pass the transmissive layer.

In one embodiment of the invention,
- the reservoir can comprise an encapsulation which - at least in an undamaged state - comprises the fluid,
- the encapsulation can be configured to be broken upon defined external influence or exposure, and
- the encapsulation, the transmissive layer and the conductive layer can be arranged relative to each other so that - in the event of a break of the encapsulation - wetting of the transmissive layer by the fluid is provideable and humidifying of the conductive layer by transmission of fluid and/or vaporous parts of the fluid through the transmissive layer is provideable.

Under the term to be broken upon defined external influence (or exposure) is understood not only the physical breaking, i.e. the bursting of the surface of the encapsulation under the influence of a force (for example triggered by bending or pressing in the surface with the fingers or hitting the surface with a kind of hammer), but any way known to the skilled person to open a surface by an external influence or exposure.

The encapsulation can also be ripped open, for example, by a pulling movement (for example, with two fingers), whereby the surface of the encapsulation can be pulled directly or on a part protruding from the surface of the encapsulation, i.e. a kind of flap.

Breaking of the encapsulation can also be achieved by scratching or cutting or piercing the encapsulation by means of an edge or blade or tip (i.e. the breaking element) or any other protruding part attached, for example, to a tilting element or a sliding element.

In a further embodiment, in the undamaged state, the encapsulation can provide separation of the fluid from the transmissive layer. The undamaged state should be understood to provide the encapsulation in an at least basically fluid-tight and vapour-tight condition. In the undamaged state, the encapsulation does not comprise any leaking.

In a further embodiment, the encapsulation can comprise a flexible wrapping, in particular the encapsulation can be made from a water vapour impermeable material, in particular comprising aluminium. The encapsulation can be provided inside of the reservoir in flexible manner.

In a further embodiment, the encapsulation can comprise or is made from a separation layer, in particular from a foil. As for example, the reservoir may be provided by a volume enclosed by walls of the reservoir and the separation layer.

In a further embodiment, the encapsulation can be configured to be broken by snapping the encapsulation. Snapping may cause a rise of pressure inside of the encapsulation which results in breaking of the encapsulation when exceeding a particular resistibility of the encapsulation.

In a further embodiment, the contact system can comprise a breaking element configured for breaking the encapsulation upon applying the defined external influence.

In a further embodiment, the breaking element can be arranged between the encapsulation and the transmissive layer.

In a further embodiment, the breaking element can be arranged in the encapsulation.

In a further embodiment, the breaking element can comprise a perforation portion configured to provide perforation of the encapsulation and arranged to perforate the encapsulation upon applying the defined external influence.

In a further embodiment, the perforation portion can comprise a tapered end, an edge or a needle.

The breaking element can be any structural body which - when interacting with the encapsulation - facilitates breaking of the encapsulation.

In a further embodiment, the transmissive layer can comprise a textile or a polymer.

In a further embodiment, the transmissive layer can be provided by a textile layer and/or by a polymeric layer.

In a further embodiment, the transmissive layer can be configured to enable transmission of vapour on the basis of the principle of partial pressure.

In a further embodiment, the transmissive layer can be configured to enable transmission of fluid by a defined transmission rate, in particular wherein the transmissive layer comprises perforations (holes) or respectively adjusted fabric and provides the characteristics of a filter.

In one embodiment, the transmissive layer can be a membrane, in particular a semipermeable membrane.

It is particularly advantageous to provide the transmissive layer and/or the conductive layer by a polymeric layer, for example, as a perforated polymer band. The perforated polymeric layer can be glued or hot-pressed directly onto the reservoir, thus saving production steps. The textile, on the other hand, must first be clamped and stretched before being installed in the contact system (e.g. electrode). Thus, the polymeric layer can be better bound to the reservoir (e.g. by gluing or melting, etc.), while the textile layer is merely pressed on.

Another advantage of using a polymeric layer instead of a textile layer to provide the transmissive layer and/or the conductive layer is that the conductive textile itself absorbs a lot of fluid, in particular water, and thus transports it to areas where no skin contact takes place. A suitable polymer does not absorb the fluid (e.g. water), so the fluid remains where it has been applied. With a conductive polymer layer, less fluid has to be used. The reservoir can be configured smaller and thus the electrode can be configured smaller and/or flatter. This in turn is more comfortable to wear on the body.

Another advantage of using a polymeric layer instead of a textile layer to provide the transmissive layer and/or the conductive layer is that the polymeric layer can be configured as a stiffer conductive porous material compared to a flexible textile, which simplifies handling, especially during production.

In addition, conductive polymer surfaces can be integrated into the contact unit during an injection molding process.

In a further embodiment, the conductive layer can be provided by the transmissive layer, wherein the transmissive layer comprises electrically conductive material.

In a further embodiment, the conductive layer can comprise an electrically conductive element, in particular electrically conductive threads or electrically conductive synthetic material, in particular made of metal or metallized synthetic material, in particular yarns.

In a further embodiment, the transmissive layer can be configured to be water vapour permeable and water impermeable with respect to liquid water.

In a further embodiment, the contact system can comprise a contacting layer configured to provide adhesion of the contact system on a surface, in particular on the skin of a person. The contact layer may comprise glue to provide the adhesion.

In a further embodiment, the contacting layer can surround the conductive layer or the contacting layer can be provided by the conductive layer.

In a further embodiment, the reservoir can be provided by a housing, in particular flexible or elastic housing. As for example, the reservoir may comprise basically stiff side walls and a flexible top wall intended to apply the external influence to break the encapsulation.

In a further embodiment, the reservoir can be provided by a flexible cover, in particular a foil. For instance, the reservoir may be provided in form of a pad to be inserted into a respective holder or receiving portion.

In a further embodiment, the fluid can comprise at least one of the following:
- a liquid, in particular a high-viscous liquid,
- a gel, in particular a hydrogel,
- a solution, in particular a water-based solution or an alcohol-based solution,
- an emulsion, and/or
- oil.

An example of an alcohol-based solution is a solution consisting of ethanol and water, wherein the proportion of ethanol can vary.

The present invention further relates to a reservoir for a contact system according to any of the preceding embodiments, the reservoir comprises
- a transmissive layer, the transmissive layer being configured to provide defined transmission of a fluid and/or of fluid-based vapour with a defined transmission rate, and
- an encapsulation which at least in an undamaged state comprises a fluid and which provides separation of the fluid from the transmissive layer,
wherein
- the encapsulation is configured to be broken upon defined external influence/exposure, and
- the encapsulation and the transmissive layer are arranged relative to each other so that - in the event of a break of the encapsulation - wetting of the transmissive layer by the fluid is provideable and transmission of the fluid and/or vaporous parts of the fluid through the transmissive layer is provideable.

In a further embodiment, the reservoir can be provided by a flexible cover.

The present invention further relates to a wearable element, in particular to a piece of clothing or garment or part thereof, for a contact system according to any of the preceding embodiments, the wearable element comprises
- a reservoir receiving segment, in particular case, and
- a conductive layer,
wherein the reservoir receiving segment and the conductive layer are arranged relative to another so that - when a reservoir according to any of the preceding embodiments is provided in the reservoir receiving segment and in the event of a break of the encapsulation of the reservoir - wetting of the transmissive layer by the fluid is provided and humidifying of the conductive layer by transmission of the fluid and/or of vaporous parts of the fluid through the transmissive layer is provideable.

The present invention further relates to a method of activating a contact system according to any one of the preceding embodiments, the method comprising the steps of
- providing the contact system so that the conductive layer is arranged close to or in contact with the skin of a person and
- successively, breaking the encapsulation by applying at least the defined external influence to the encapsulation.

The method to activate the contact system provides the advantage that a user of the contact system is enabled to activate the electrode after attaching the electrode, e.g. on the user's skin.

### BRIEF DESCRIPTION OF THE DRAWINGS

The invention is illustrated in more detail below, purely by way of example, with reference to working examples shown schematically in the drawing. Identical elements are labelled with the same reference numerals in the figures. The described embodiments are generally not shown true to scale and they are also not to be interpreted as limiting the invention.
Figure 1 shows an exemplary embodiment of a contact system according to the invention;
Figure 2 shows an exemplary embodiment of a contact system according to the invention;
Figures 3a and 3b show an exemplary embodiment of a contact system according to the invention;
Figures 4a and 4b show an exemplary embodiment of a contact system according to the invention;
Figures 5a and 5b show an exemplary embodiment of a contact system according to the invention;
Figures 6a and 6b show an exemplary embodiment of a contact system according to the invention;
Figure 7 shows an exemplary embodiment of a contact system according to the invention;
Figure 8 shows an exemplary embodiment of a contact system according to the invention; and
Figures 9a and 9b show an exemplary embodiment of a contact system according to the invention provided with a wearable.

### DETAILED DESCRIPTION OF THE DRAWINGS

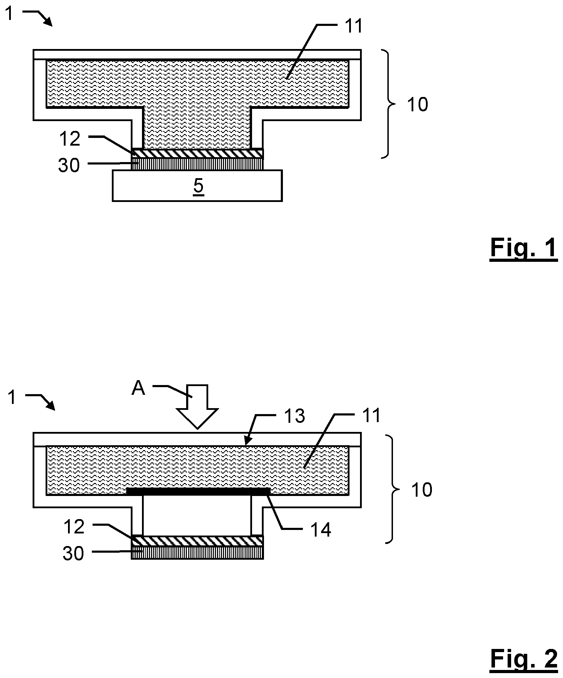

**Figure 1** shows an embodiment of a contact system 1 according to the invention. The contact system 1 comprises a reservoir 10 and a conductive layer 30.

The reservoir 10 further comprises a fluid 11 and a transmissive layer 12 which is configured to provide defined transmission of vapour and/or vapour-like substance and/or vaporous substance. In particular, the transmissive layer 12 is provided as a layer being permeable for fluid vapour but being impermeable for liquid fluid, in particular when the fluid is water or a water-based solution. The transmissive layer 12 is preferably designed so that a defined amount of vapour transmits the layer 12 in a defined time period.

In one embodiment, the transmissive layer 12 is provided as a layer being permeable for the fluid in a way to provide passing of a defined amount of the fluid in a defined time period. Here, the transmissive layer 12 can provide the functionality of a filter.

The transmissive layer 12 can be provided by a respectively permeable textile or by a perforated polymeric layer.

The contact system 1 is arranged on an object 5. The object 5 can be the skin of a person. The contact system 1 is arranged so that the conductive layer 30 is in contact with the object 5. In particular, the contact system 1 adheres on the object 5 by means of fixing means which may be provided by particular glue and/or by a respective contacting layer (not shown).

The (electrically) conductive layer 30 is configured to receive and transmit electrical signals which are provided by the object 5. By that, the contact system 1 provides a system to obtain electrical signals from the object. In particular, in case the object 5 is represented by the skin of a person, the contact system 1 provides transmission of electrical signals generated by particular organs, like the heart of the person, and/or transmitted by particular nerves or tissue. Such electrical signals can be detected on the skin of the person.

Alternatively or additionally the conductive layer 30 can be configured to transmit electrical signals to the object 5. Such configuration can for instance be used to provide stimulation of the object 5, e.g. of a muscle.

An electric conductor can be connected to the conductive layer 30 in order to transmit the collected signals to a respective receiving unit, e.g. to an ECG (electrocardiogram) device, or to the object 5.

The transmissive layer 12 and the conductive layer 30 are arranged relative to each other so that humidifying of the conductive layer 30 by transmission of vaporous parts of the fluid 11 or of parts of the fluid through the transmissive layer 12 is provided.

Due to the arrangement and designs of the layers 12 and 30 the conductive layer 30 can be kept humid over a comparatively long time period. This is provided by defined diffusion of the fluid 11 in vapour-like form, i.e. out of the reservoir by passing the transmissive layer 12 and wetting the conductive layer 30. In other words, there can be provided continuous transmission of parts of the fluid, wherein the parts of the fluid can be provided as vapour from the fluid or gaseous droplets of the fluid (e.g. of a liquid).

In particular, the configuration of the transmissive layer 12 is adjusted with respect to the type of fluid 11 so that the wetting of the conductive layer 30 is provided as desired. In particular, the transmissive layer 12 comprises particularly designed perforations or respectively tight weaving or knitting.

As a result of the wetting of the conductive layer 30, electrical conductivity between the object 5 and the conductive layer 30 and/or inside of the conductive layer 30 is provided and can be maintained over a comparatively long period of time due to slow and dosed diffusion or permeation of the fluid.

Figure 2 shows a further embodiment of a contact system 1 according to the invention. In contrast to the embodiment of figure 1, the reservoir 10 of the contact system 1 here comprises an encapsulation 13. The encapsulation 13 comprises the fluid 11. In the shown embodiment, the encapsulation 13 is provided by the walls of the reservoir as well as by a separation layer 14. The separation layer 14 provides a separation of the fluid 11 from the transmissive layer 12 in order to prevent from unwanted wetting of the transmissive layer 12 and/or the conductive layer 30. In other words, the separation layer 14 is configured to be tight, in particular gas-tight, with respect to the fluid 11 and acts as a seal.

The separation layer 14 can be a foil or can be manufactured from any material which provides respective sealing properties.

The encapsulation 13, in particular the separation layer 14, is configured to be broken upon defined external influence or exposure. In the shown embodiment, the properties of the encapsulation 13 allow to intentionally damage the encapsulation 13 by applying a particular force to the reservoir 10 or the encapsulation 13. As shown with arrow A, a force can be applied on top of the contact system 1, i.e. on the top wall of the encapsulation 13, which causes a respective force or pressure on the separation layer 14. The separation layer 14 is configured to break upon exposure of the a defined force or stress.

With such configuration, a contact system 1 can be provided which can be activated on demand, i.e. when pressing on the contact system 1 from top. Activation in this context should be understood to start and provide wetting of the transmissive layer 12 and/or the conductive layer 30 as described above.

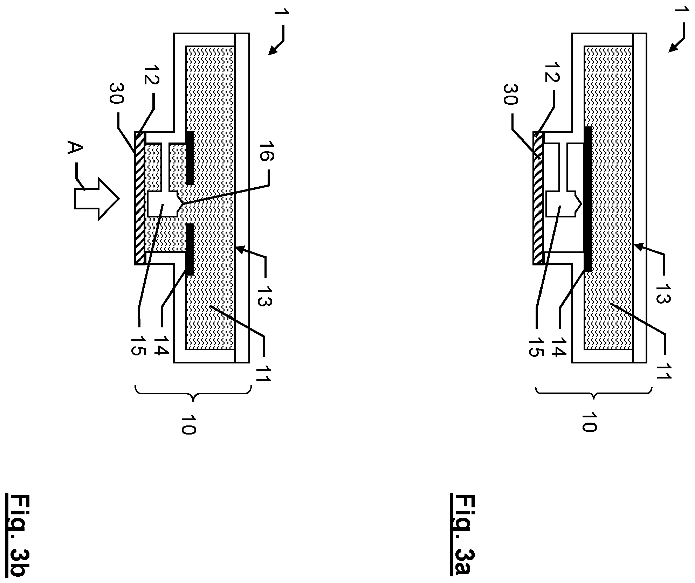

Figures 3a and 3b show a further embodiment of a contact system 1 according to the invention. In contrast to the embodiment of figure 2, the contact system 1 comprises a breaking element 15. Furthermore, here the conductive layer 30 is provided by the transmissive layer 12, wherein the transmissive layer 12 comprises electrically conductive material. However, even if it is shown here like this, it is to be understood that the arrangement of the breaking element 15 as shown and the combination of the transmissive the conductive layer may be implemented independently from another in respective embodiments.

In particular, the transmissive layer 12 comprises conductive parts which provide the conductive layer. In particular, the combined layers are provided by a textile having electrically conductive threads, e.g. the electrically conductive threads are woven in the textile.

The breaking element 15 is arranged between the encapsulation 13, in particular the separation layer 14, and the combined transmissive the conductive layer. Hence the breaking element 15 is arranged in a bottom part of the electrode 1, which part is intended to be directed towards the object in use.

The breaking element 15 comprises a perforation portion (sharp edge) on its top side directed towards the separation layer 14 for damaging, perforating or breaking the separation layer 14. In an alternative embodiment the perforation portion can be embodied as a needle-like extension for damaging, perforating or breaking the separation layer 14.

The contact system 1 according to the present embodiment is provided as an embodiment intended to be activated before arranging the contact system 1 on the object. Thanks to the breaking element 15, activation can be realised by applying comparatively low pressure or force from the bottom side as depicted by the arrow A. As can be seen in figure 3b, the separation layer is broken after applying a defined external pressure to the breaking element 15. Hence, activation can be performed easier by applying less pressure.

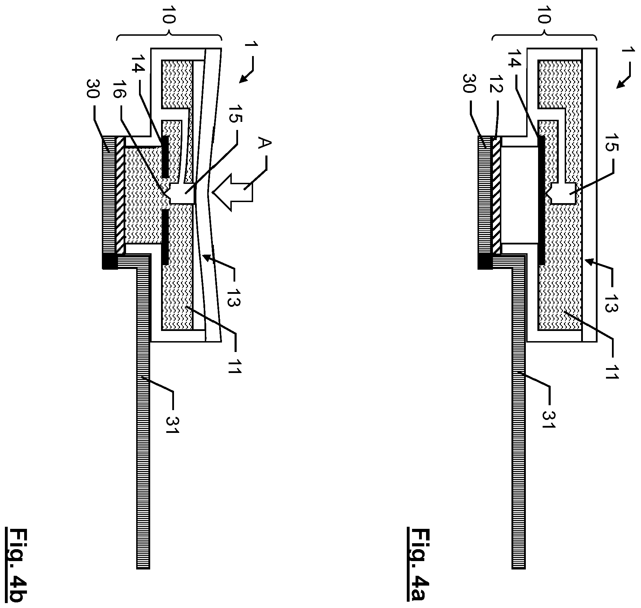

Figures 4a and 4b show a further embodiment of a contact system 1 according to the invention. In contrast to the embodiment of figures 3a and 3b, the breaking element 15 is arranged on the opposite side of the separation layer 14. Here, the breaking element 15 is arranged inside of the encapsulation 13.

The breaking element 15 comprises a perforation portion 16 configured to provide perforation of the encapsulation 13, in particular of the separation layer 14, and arranged to perforate the separation layer 14 upon applying the defined external influence on top of the contact system 1.

As can be seen from figure 4b, the activation force can be applied on the top surface of the encapsulation as shown with arrow A. The top surface is made elastic to provide bending of the surface. By that, when applying the activation force, the top surface of the encapsulation 13 first touches the breaking element 15 and, second, moves the breaking element 15 towards the separation layer 14 to provide interaction of the perforation portion 16 with the separation layer 14. In the course of onward moving of the breaking element 15, the separation layer 14 becomes intentionally damaged, i.e. perforated, broken or cracked. Consequently, the sealing properties of the separation layer 14 are lost and the fluid 11 can then pass the separation layer 14 to get in touch with the transmissive layer 12.

As described above, after damaging the separation layer 14, the fluid 11 wets and passes or permeates the transmissive layer 12. By that, wetting of the conductive layer 30 is started and/or provided.

Activation (e.g. triggering the breaking mechanism) from above provides the advantage that the contact system 1 (e.g. the electrode) can also be activated if activation was forgotten before the contact system 1 was attached to the skin (e.g. sticking the electrode on). A healthcare provider or a patient can thus activate the contact system 1 (e.g. the electrode) retrospectively.

The contact system 1 also comprises an electrical conductive wire 31 which is connected to the conductive layer 30. The electrical conductive wire 31 provides connection of the electrode 1 to an external device like an ECG device.

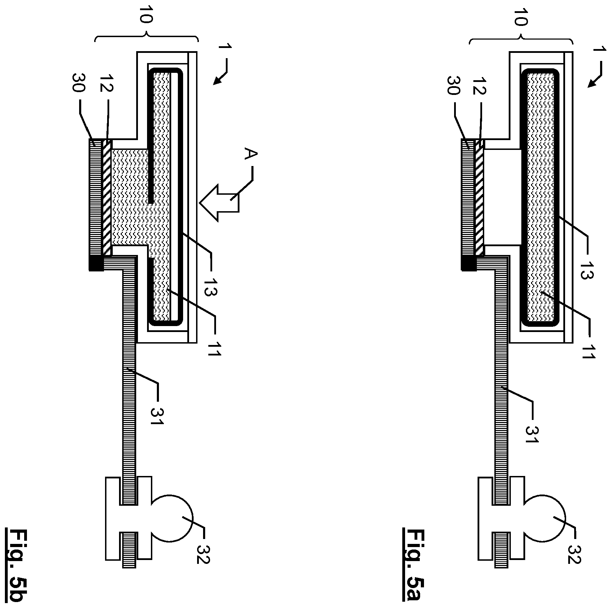

Figures 5a and 5b show a further embodiment of a contact system 1 according to the invention. In contrast to the embodiment of figure 2, the encapsulation 13 is here provided by a flexible cover which, in an undamaged state (see figure 5a), encloses the fluid 11. Hence, the encapsulation 13 is provided in form of a pad having the fluid 11 inside.

Furthermore an electrical conductor 31 is connected to the conductive layer 30. An interface or pin 32 is connected to the conductor 31 in order to provide easy connection with a measuring or stimulation device.

The pad 13 is configured to be broken by applying a pressure on the top of the electrode 1 (see figure 5b). The pad 13 preferably gets damaged by the edges provided in the housing of the reservoir 10.

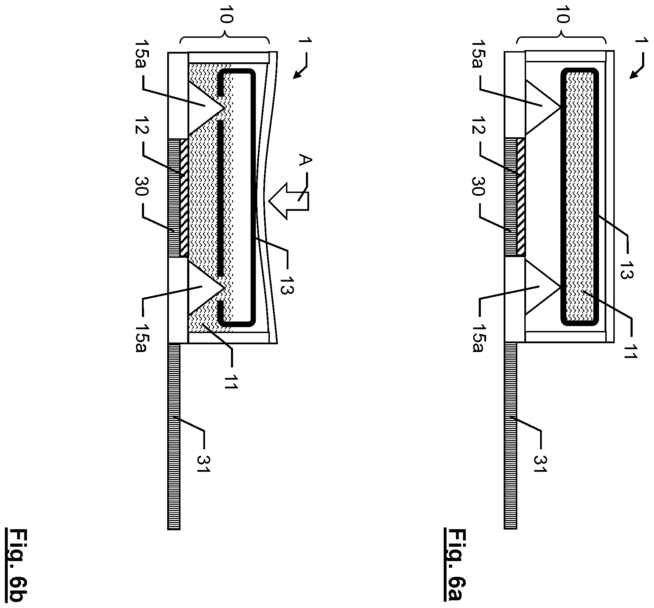

Figures 6a and 6b show a further embodiment of a contact system 1 according to the invention. The contact system 1 comprises a reservoir 10 and a conductive layer 30.

The reservoir 10 comprises a transmissive layer 12 which is configured to provide defined permeation of vapour and/or vapour-like substance and/or vaporous substance and comprises an encapsulation 13 which encloses a fluid 11. The transmissive layer 12 is provided as a layer being permeable for fluid vapour but being impermeable for liquid fluid, in particular when the fluid is water or a water-based solution. The transmissive layer 12 is designed so that a defined amount of vapour transmits the layer 12 in a defined time period.

The transmissive layer 12 can be provided by a respectively permeable textile or by a perforated polymeric layer.

The encapsulation 13 is provided by a fluid-tight sheath (cover) which provides gas-tight enclosure of the fluid 11. The contact system 1 also comprises at least two breaking elements 15a which here are provided in form of pyramidal elevations with peaked ends. The encapsulation 13 is arranged on top of the breaking elements 15a. The cover of the encapsulation 13 is configured so that the encapsulation 13 is not damaged by the breaking elements 15a and maintains its fluid-tight property in an inactivated state, i.e. in particular as long as there is not applied an external activating force A from top.

Figure 6b shows the contact system 1 after activation by applying a pressure on a top wall of the reservoir 10. By applying the pressure in a direction according to arrow A, the encapsulation 13 is pressed against the breaking elements 15a and is cracked by interaction with the breaking elements 15a. As a result, the fluid 11 flows out of the encapsulation 13 and contacts the transmissive layer 12. The fluid 11 provides wetting of the transmissive layer 12 and vapour-like parts of the fluid passes through the transmissive layer 12 and cause wetting of the contact layer 30.

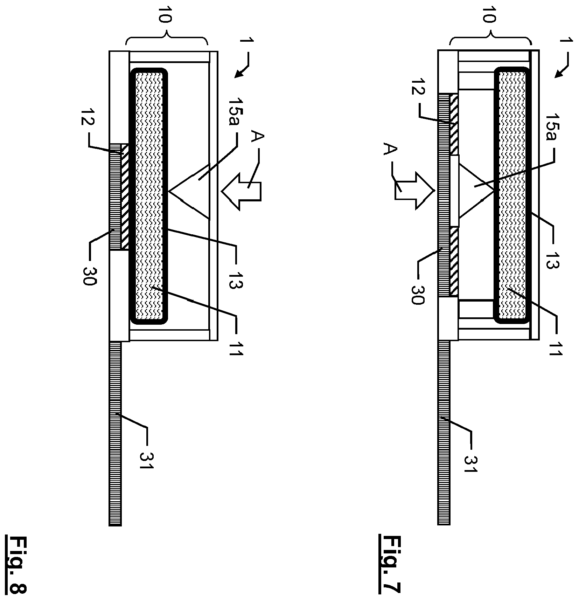

Figures 7 and 8 show further embodiments of a contact system 1 according to the invention. Both embodiments comprise an activation system which is provided by an encapsulation 13 and a breaking element 13 for perforating the encapsulation 13 upon applying at least a defined activation force (depicted by arrow A). The embodiments differ in the arrangement of the breaking element 15a and in size and configuration of the transmissive and/or the conductive layers.

According to figure 7 the breaking element is arranged between the encapsulation 13 and the transmissive layer 12 and the electrode 1 is intended to be activated by pressing from below, i.e. on side of the contacting layer before arranging the electrode 1 on the skin of a person. The transmissive layer 12 is provided as a ring which surrounds the breaking element 15a. Alternatively, the transmissive layer 12 is provided by particular transmissive segments.

The conductive layer 30 is built to entirely covering the transmissive layer 12 in order to provide advantageous wetting and improved electrical conductivity to the skin.

According to figure 8 the encapsulation 13 is arranged between the breaking element 15a and the transmissive layer 12. Here, the electrode 1 is intended to be activated by pressing from top, i.e. directly on the top wall of the reservoir 10 on which the breaking element 15a is mounted. The electrode 1 of figure 8 can easily be activated after being arranged on the skin of a person.

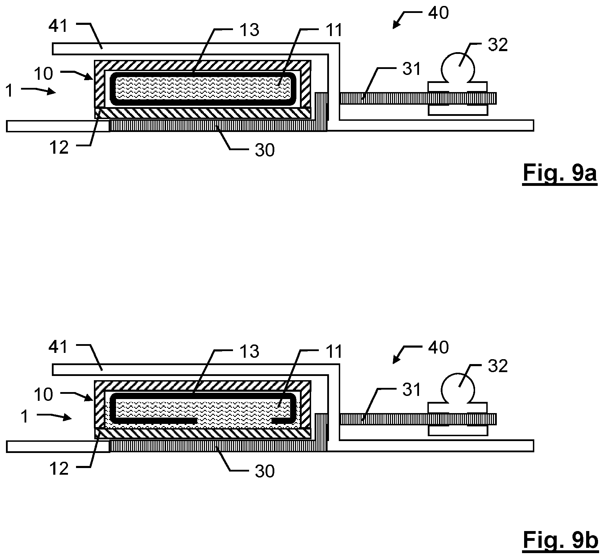

Figures 9a and 9b show a further embodiment of a contact system 1 according to the invention. The contact system 1 comprises a reservoir 10 which is provided by a flexible cover, in particular by a foil or textile. Here, the reservoir 10 is preferably embodied as a pad.

The reservoir 10 comprises an encapsulation 13. A fluid 11 is provided inside of the encapsulation 13. The encapsulation 13 is configured to be tight with respect to the contained fluid 11. Moreover, the encapsulation 13 is configured to be breakable upon exposure of an external force and, by that, to provide outflowing of the fluid (see figure 9b), i.e. the encapsulation 13 can be broken when pressing on the encapsulation 13 and/or when snapping, squeezing or cracking the encapsulation 13.

At least a part of the reservoir 10 is made from a transmissive material and by that provides a transmissive layer 12. In the shown embodiment, the bottom side (facing the conductive layer 30) provides the transmissive layer 12. The transmissive layer 12 is moistened when the encapsulation 13 is broken.

The contact system 1 also comprises a conductive layer 30 which is connected with a wire 31 to provide electrical conductivity to and from a connecting pin 32.

The conductive layer 30 is provided by a wearable element 40, i.e. by a piece of clothing. In particular, the conductive layer 30 is provided by electrical conductive threads which are woven in the wearable element 40. Alternatively, the conductive layer 30 can be provided by an additional textile or polymeric layer which is glued or sewn on the wearable element 40.

The wearable element 40 comprises a reservoir receiving segment 41, in particular case or bag. The bag 41 is configured to receive the reservoir 10. In particular, the reservoir 10 can be inserted into the reservoir receiving segment 41. The encapsulation 13 is preferably broken after inserting in order to provide wetting of the conductive layer 30 on demand.

The reservoir receiving segment 41 and the conductive layer 30 are arranged relative to another so that - when a reservoir 10 is provided in the reservoir receiving segment 41 and in the event of a break of the encapsulation 13 of the reservoir 10 - wetting of the transmissive layer 12 by the fluid 11 is provided and humidifying of the conductive layer 30 by transmission of vaporous parts of the fluid 11 through the transmissive layer 12 is provided.

The reservoir 10 can be removed from the reservoir receiving segment 41 when the fluid 11 is consumed and wetting of the conductive layer 30 can no longer be provided. Moreover, a further (new, undamaged) reservoir 10 (pad) can then be inserted into the bag 41 to continue wetting of the conductive layer 30.

The wearable element 40 can be provided as a shirt or pants or may be any other wearable component intended to be worn and having contact with the body of a person.

Although the invention is illustrated above, partly with reference to some preferred embodiments, it must be understood that numerous modifications and combinations of different features of the embodiments can be made. All of these modifications lie within the scope of the appended claims.

## Claims

1. Contact system (1), in particular electrode, for providing an electric contact to the skin of a person, the contact system (1) comprising
• a reservoir (10) comprising a fluid (11) and
• a conductive layer (30),
**characterised in that**
• the reservoir (10) comprises a transmissive layer (12), the transmissive layer (12) being configured to provide defined transmission of the fluid and/or of fluid-based vapour with a defined transmission rate, and
• the transmissive layer (12) and the conductive layer (30) are arranged relative to each other so that humidifying of the conductive layer (30) by transmission of vaporous parts of the fluid through the transmissive layer (12) is provideable.

2. Contact system (1) according to claim 1, wherein
• the reservoir (10) comprises an encapsulation (13) which at least in an undamaged state comprises the fluid (11),
• the encapsulation (13) is configured to be broken upon defined external influence and
• the encapsulation (13), the transmissive layer (12) and the conductive layer (30) are arranged relative to each other so that - in the event of a break of the encapsulation (13) - wetting of the transmissive layer (12) by the fluid is provideable and humidifying of the conductive layer (30) by transmission of fluid and/or vaporous parts of the fluid (11) through the transmissive layer (12) is provideable.

3. Contact system (1) according to claim 2, wherein
in the undamaged state, the encapsulation (13) provides separation of the fluid (11) from the transmissive layer (12).

4. Contact system (1) according to claim 2 or 3, wherein
the encapsulation (13)
• comprises a flexible wrapping, in particular is made from a material comprising aluminium, and/or
• comprises or is made from a separation layer (14), in particular foil, and/or
• is configured to be broken by snapping the encapsulation (13).

5. Contact system (1) according to anyone of the claims 2 to 4, wherein the contact system comprises a breaking element (15) configured for breaking the encapsulation (13) upon applying the defined external influence.

6. Contact system (1) according to claim 5, wherein
• the breaking element (15) is arranged between the encapsulation (13) and the transmissive layer (12), or
• the breaking element (15) is arranged in the encapsulation (13).

7. Contact system (1) according to anyone of the claims 5 or 6, wherein the breaking element (15) comprises a perforation portion (16) configured to provide perforation of the encapsulation (13) and arranged to perforate the encapsulation (13) upon applying the defined external influence, in particular wherein the perforation portion (16) comprises a tapered end, an edge or a needle.

8. Contact system (1) according to anyone of the preceding claims, wherein the transmissive layer (12)
• comprises a textile or a polymer, and/or
• is provided by a textile layer and/or by a polymeric layer, and/or
• is configured to be water vapour permeable and water impermeable with respect to liquid water.

9. Contact system (1) according to anyone of the preceding claims, wherein the conductive layer (30) is provided by the transmissive layer (12), wherein the transmissive layer (12) comprises electrically conductive material.

10. Contact system (1) according to anyone of the preceding claims, wherein the conductive layer (30) comprises an electrically conductive element, in particular electrically conductive threads or electrically conductive synthetic material, in particular made of metal or metallized synthetic material, in particular yarns.

11. Contact system (1) according to anyone of the preceding claims, wherein the reservoir (10)
• is provided by a housing, in particular flexible or elastic housing, or
• the reservoir (10) is provided by a flexible cover, in particular a foil.

12. Contact system (1) according to anyone of the preceding claims, wherein the fluid (11) comprises at least one of the following:
• a liquid, in particular a high-viscous liquid,
• a gel, in particular a hydrogel,
• a solution, in particular a water-based solution or an alcohol-based solution,
• an emulsion, or
• oil.

13. Reservoir (10) for a contact system according to any of the preceding claims, the reservoir (10) comprises
• a transmissive layer (12), the transmissive layer (12) being configured to provide defined transmission of a fluid and/or of fluid-based vapourwith a defined transmission rate , and
• an encapsulation (13) which at least in an undamaged state comprises a fluid (11) and which provides separation of the fluid from the transmissive layer (12),
wherein
• the encapsulation (13) is configured to be broken upon defined external influence, and
• the encapsulation (13) and the transmissive layer (12) are arranged relative to each other so that - in the event of a break of the encapsulation (13) - wetting of the transmissive layer (12) by the fluid is provideable and transmission of the fluid and/or vaporous parts of the fluid through the transmissive layer (12) is provideable,
in particular wherein the reservoir (10) is provided by a flexible cover.

14. Wearable element (40) for a contact system (1) according to any of the claims 1 to 12, the wearable element (40) comprises
• a reservoir receiving segment (41), in particular case, and
• a conductive layer (30),
wherein
the reservoir receiving segment (41) and the conductive layer (30) are arranged relative to another so that - when a reservoir (10) according to claim 13 is provided in the reservoir receiving segment (41) and in the event of a break of the encapsulation (13) of the reservoir (10) - wetting of the transmissive layer (12) by the fluid is provided and humidifying of the conductive layer (30) by transmission of the fluid and/or of vaporous parts of the fluid through the transmissive layer (12) is provideable.

15. Method of activating a contact system (1) according to any one of the claims 2 to 7, the method comprising the steps of
• providing the contact system (1) so that the conductive layer (30) is arranged close to or in contact with the skin of a person and
• successively, breaking the encapsulation (13) by applying at least the defined external influence to the encapsulation (13).
